(19) Europäisches Patentamt
European Patent Office
Office européen des brevets

(11) Veröffentlichungsnummer: **0 005 758**
**B1**

(12) **EUROPÄISCHE PATENTSCHRIFT**

(45) Veröffentlichungstag der Patentschrift: **18.05.83**

(21) Anmeldenummer: **79101468.1**

(22) Anmeldetag: **14.05.79**

(51) Int. Cl.³: **C 07 J 5/00,** C 07 J 63/00, C 07 J 1/00, A 61 K 31/565 //C07J31/00, C07J33/00, C07J41/00

# (54) Verfahren zum Aufbau der Hydroxyacetyl-Seitenkette von Steroiden des Pregnan-Typs, neue 21-Hydroxy-20-oxo-17alpha-pregnan-Verbindungen und pharmazeutische Präparate enthaltend dieselben.

(30) Priorität: **26.05.78 CH 5778/78**

(43) Veröffentlichungstag der Anmeldung: **12.12.79 Patentblatt 79/25**

(45) Bekanntmachung des Hinweises auf die Patenterteilung: **18.05.83 Patentblatt 83/20**

(84) Benannte Vertragsstaaten: **AT BE CH DE FR GB IT NL SE**

(56) Entgegenhaltungen:
**CH - A - 241 645**
**DE - A - 2 823 239**

**TETRAHEDRON, Band 31, Nr. 17, September 1975, Oxford (GB) J. R. BULL, A. TUINMAN, "An efficient method for converting 17 oxo into 17-acetyl steroids" Seiten 2151—2155**

(73) Patentinhaber: **CIBA-GEIGY AG**
**Patentabteilung Postfach**
**CH-4002 Basel (CH)**

(72) Erfinder: **Biollaz, Michel, Dr.**
**Morystrasse 2**
**CH-4125 Riehen (CH)**

(56) Entgegenhaltungen:
**CHEMICAL ABSTRACT, vol. 91, nr. 3, 22-07-1974, Columbus, Ohio, USA Seite 309, zweite Spalte Zusammenfassung 13269s**

**TETRAHEDRON LETTERS, Band 26, Juni 1972, Oxford (GB) KATSUYUKI OGURA et al. "A new synthetic approach to alfa-hydroxyaldehydes using methyl methylthiomethyl sulfoxide", Seiten 2681—2684.**

**EP 0 005 758 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zum Aufbau der Hydroxyacetyl-Seitenkette von Steroiden des Pregnan-Typs, neue 21-Hydroxy-20-oxo-17$\alpha$-pregnan-Verbindungen und pharmazeutische Präparate enthaltend dieselben.

Die vorliegende Erfindung betrifft in erster Linie ein neuartiges allgemeines Verfahren zum Aubau einer $\alpha$- oder $\beta$-orientierten Hydroxyacetyl-Seitenkette von Steroiden des Pregnan-Typs (einschliesslich der D-Homo-verbindungen), das dadurch gekennzeichnet ist, dass man einen entsprechenden Steroidcarbaldehyd unter vorübergehen- dem Schutz übriger gegebenenfalls vorhandener Oxogruppen mit Formaldehyd-dimethylmercaptal-S-oxid in Form eines Alkalimetallsalzes davon, und danach, gewünschtenfalls nach Regenerierung der übrigen Oxogruppen, mit einem stark sauren hydrolysierenden Mittel behandelt und einen erhaltenen Endstoff gewünschtenfalls acyliert.

Zahlreiche 21-Hydroxy-20-oxo-17$\beta$-pregnan-Verbindungen sind bekannt als therapeutisch wertvolle Substanzen mit der Wirkung natürlicher Corticoide, d.h. der Hormone der Nebennierenrinde, oder können als Zwischenprodukte zur Herstellung solcher Wirkstoffe dienen. Bisher wurden diese Verbindungen vorwiegend durch die Einführung einer gegebenenfalls acylierten Hydroxylgruppe in die 21-Stellung einer entsprechenden 21-unsubstituierten 20-Oxo-pregnan- Verbindung hergestellt. Die Einführung erfolgt üblicherweise mehrstufig, meistens mittels einer direkten oder indirekten Halogenierung mit nachfolgendem Austausch des Halogens gegen eine Acyloxygruppe. Verschiedene Varianten dieser Methode sind in C. Djerassi (Editor): Steroid Reactions, Seite 578—586; Holden-Day, San Fransisco, 1963, übersichtlich zusammengefasst; ihr Nachteil besteht jedoch darin, dass sie lediglich für die thermodynamisch stabilere 20-Oxoverbindungen der 17$\beta$-Reihe geeignet sind, wogegen die entsprechenden 17$\alpha$-isomeren Verbindungen sich unter den Reaktionsbedingungen mindestens teilweise in die erstgenannten umlagern. Man kann auch von einer Steroid-17-carbonsäure ausgehen, welche dann in Form des entsprechenden Säurechlorids mit Diazomethan das entsprechende Diazoketon (eine 21-Diazo-20-oxopregnan-Verbindung) liefert und dieses durch Behandlung mit einer gewünschten Carbonsäure, vorzugsweise Essigsäure, den verlangten Endstoff — eine 21-Acyloxy-20-oxo-pregnan-Verbindung — ergibt. Auch diese Methode (vgl. die oben zitierte Uebersicht) ist praktisch nur für Isomere der 17$\beta$-Reihe geeignet, da die $\alpha$-Carbonsäuren nur auf umständlichen Umwegen herstellbar sind. Ganz allgemein kann man sagen, dass für diese thermodynamisch weniger stabilen $\alpha$-Isomeren kein zuverlässiger systematischer Zugang vorhanden ist, obwohl solche Verbindungen potentiell von pharmakologischem Interesse sind.

Das erfindungsgemässe Verfahren dagegen ist von den geschilderten Nachteilen frei und kann zu Produkten sowohl der $\beta$- wie auch der $\alpha$-Konfiguration führen: erstens sind die als Ausgangsstoffe verwendeten Steroidcarbaldehyde in beiden Konfigurationen leicht zugänglich, wie noch weiter unten näher beschrieben wird, zweitens werden diese Ausgangsstoffe durch Umwandlung von entsprechenden 17- bzw. 17a-Oxosteroiden hergestellt, also von Verbindungen, die auch durch totalsynthetische Methoden gut zugänglich sind. (Der Umstand, dass die Totalsynthese von Steroiden in immer zunehmendem Masse an Wichtigkeit gewinnt, macht das erfindungsgemässe Verfahren als einen sehr vorteilhaften totalsynthetischen Zugang zu den Steroiden mit der Hydroxyacetyl-Seitenkette der Pregnan-Steroide besonders wertvoll.)

Das Reagens für die erste Stufe des erfindungsgemässen Verfahrens ist an sich bekannt und seine Zubereitung wurde beschrieben, vgl. K. Ogura und G. Tsuchihashi, Tetrahedron Letters *1972*, No. 26, S. 2681—2684. In dieser Publikation wurde auch die Möglichkeit der Anwendung des Reagens zur Synthese von einzelnen einfachen $\alpha$-Hydroxyaldehyden, angedeutet. Als eine vereinzelte Ausnahme von diesem normalen Verlauf betrachten die Autoren die von ihnen auf Seite 2683, Gleichung 5, erwähnte Umwandlung von Ph—CH(OH)—CH($SCH_3$)$SOCH_3$ zum Ph—CO—$CH_2OH$ mit konzentrierter Salzsäure. Dieses anomale Resultat kann man mit hoher Wahrscheinlichkeit dem aromatischen Charakter des Phenyls zuschreiben, welcher die Verschiebung der Carbonyl-Doppelbindung der Aldehydogruppe aus der allylischen $\beta$, $\gamma$-Stellung in die stabile konjugierte $\alpha$, $\beta$-Stellung unter den sauren, enolisierenden Bedingungen stark unterstützen wird. — Im vorliegenden Fall der erfindungsgemässen gesättigten Produkte liegt kein solcher fördernder Einfluss vor, im Gegenteil, es war zu befürchten, dass das notwendige stark saure Milieu nicht nur zur Enolisierung, sondern damit auch zwangsläufig zur unerwünschten Isomerisierung der Seitenkette in die thermodynamisch stabilere Konfiguration führen würde. Dass dies nicht der Fall ist, und dass im Endstoff die entscheidende Konfiguration des Ausgangsstoffes erhalten bleibt, gehört zu den wichtigsten, völlig unerwarteten Vorteilen das erfindungsgemässen Verfahrens. — Uebrigens ist die Tatsache, dass die erfindungsgemässe Umsetzung ohne partielle Isomerisierung der entscheidenden Konfiguration in 17-Stellung verläuft und somit eine problemlose Isolierung des Endstoffes ermöglicht, umso erstaunlicher, als durch die Analogie mit dem bekannten Stand der Technik, z.B. dem oben angegebenen, zu erwarten war, dass die Anwendung eines stark basischen Milieu in der ersten Stufe, genauso wie die des stark sauren Milieu in der zweiten Stufe, die Enolisierung und die damit verbundene 17-Isomerisierung zur Folge haben würde.

Das erfindungsgemäss in der ersten Verfahrensstufe eingesetzte Reagens wird zubereitet, indem man das Formaldehyddimethylmercaptal-S-oxid ($CH_3.S.CH_2SO.CH_3$) mit einer Hydrocarbyl-Alkalimetall-Verbindung in einem aprotischen Lösungsmittel umsetzt. Das bevorzugte Alkalimetall ist Lithium; man kann jedoch aber auch Natrium oder Kalium verwenden. Der Hydrocarbylrest in der Organometall-Verbindung ist zum Beispiel ein Aryl, insbesondere ein monocyclisches Aryl, wie vorzugsweise Phenyl, oder

ein Niederalkyl, insbesondere ein primäres Alkyl, wie ganz speziell Butyl. Bevorzugte Organometall-Verbindungen (Hydrocarbyl-Alkalimetall-Verbindungen) sind Phenyllithium und vor allem Butyllithium. Als aprotische Lösungsmittel für die Zubereitung des Reagens eignen sich insbesondere Kohlenwasserstoffe und Aether, vorzugsweise solche, deren Siedepunkt unter 125°C liegt. Unter Kohlenwasserstoffen sind bevorzugt gesättigte aliphatische oder cycloaliphatische Kohlenwasserstoffe, wie insbesondere Pentan, Hexan und Heptan, beziehungsweise Cyclohexan, weiter kommen auch aromatische Kohlenwasserstoffe, wie insbesondere Benzol, in Betracht. Bevorzugte Aether sind einerseits aliphatische Aether, insbesondere Diäthyläther und 1,2-Dimethoxyäthan, anderseits cyclische Aether, wie Tetrahydropyran, Dioxan und vor allem Tetrahydrofuran. Man kann auch Kombinationen von zwei oder mehreren dieser Lösungsmittel verwenden. Die Zubereitung des Reagens erfolgt üblicherweise durch Zufügen einer Lösung der Organometallverbindung zu einer Lösung von Formaldehyd-dimethylmercaptal-S-oxid bei einer Temperatur unterhalb 0°, üblicherweise bei etwa —25° bis etwa —15°; ein Ueberschuss an der Organometall-Verbindung ist vornehmlich zu vermeiden. Diese Lösung des in situ zubereiteten Reagens versetzt man dann mit dem umzusetzenden Steroid-aldehyd, üblicherweise in Form einer Lösung in einem der genannten Lösungsmittel. Die Reaktions-temperatur bewegt sich zwischen etwa —50° bis +50°, vorzugsweise im Bereich von etwa —20° bis 0°. das primäre Reaktionsgemisch wird nach beendeter Reaktion, d.h. je nach den jeweiligen Bedingungen nach etwa 1 bis 24 Stunden, mit Wasser zersetzt un in üblicher Weise weiterverarbeitet. — es ist empfehlenswert, die üblichen Massnahmen für die Arbeit mit Organometall-Verbindungen sowohl während der Zubereitung des Reagens wie auch der eigentlichen Kondensation einzuhalten, z.B. Spuren von protischen Lösungsmitteln, wie insbesondere von Alkoholen und Wasser, auch in Form von Luftfeuchtigkeit, zu vermeiden, und unter Atmosphäre eines inerten Gases, wie insbesondere unter Argon, zu arbeiten. — Diese Umsetzung führt zu einem Produkt, dessen Seitenkette durch die Partial-formel

$$\begin{array}{c} \quad 20 \qquad 21 \\ -\underset{\displaystyle OH}{\underset{|}{CH}}-\underset{\displaystyle SCH_3}{\underset{|}{CH}}-SOCH_3 \end{array}$$

charakterisiert ist. Da in dieser Seitenkette sowohl das 20-ständige C-Atom (durch die Hydroxylgruppe) wie auch das 21-ständige C-Atom (durch die beiden schwefelhaltigen Gruppen) asymmetrisch sind, d.h. entweder die absolute Konfiguration R oder S haben können, ist das wirkliche Reaktionsprodukt ein Gemisch von mehreren der vier möglichen Stereoisomeren (d.h. 20R, 21R-; 20R, 21S-; 20S, 21R- und 20S, 21S-), welche in wechselndem, unspezifischem Verhältnis vorhanden sein können. Dies ist jedoch von keinem Nachteil für die Weiterverarbeitung in der zweiten erfindungsgemässen Verfahrensstufe, da alle vier Isomeren denselben Endstoff ergeben; deshalb erübrigt sich auch jegliche Auftrennung des Isomerengemisches.

Während der Umsetzung gemäss der ersten Reaktionsstufe ist es vorteilhaft, die übrigen im Ausgangsstoff gegebenenfalls vorhandenen Oxogruppen, wie insbesondere die 3-Oxogruppe, aber auch z.B. die 11-, 12-, 18- oder 19-Oxogruppe, gegen eine unerwünschte Reaktion vorübergehend zu schützen. Zum vorübergehenden Schutz der Oxogruppen verwendet man konventionelle Massnahmen: z.B. wird die Oxogruppe zur Hydroxylgruppe reduziert und nachträglich durch Oxidation regeneriert, oder insbesondere in ein basenbeständiges funktionelles Derivat übergeführt und nachträglich in üblicher Weise, z.B. mittels Hydrolyse, freigesetzt. (Vornehmlich kann der Ausgangsstoff solche Oxogruppen bereits von seiner vorangehenden Herstellung her in einer solchen geschützten Form enthalten.)

Als basenbeständige funktionelle Derivate zum Schutz der Oxogruppen kommen insbesondere Ketale und Acetale, bzw. Thioketale und Thioacetale, in Betracht. Unter den ersteren sind solche bevorzugt, die sich von Niederalkanolen, wie Methanol oder Aethanol, und insbesondere von 1,2- oder 1,3-Niederalkandiolen, wie 1,2- oder 1,3-Propandiol, 1,2-oder 2,3-Butandiol, und vor allem Aethylenglykol, ableiten. Als Thioketale bzw. Thioacetale kommen insbesondere diejenigen in Betracht, die sich von Schwefelanalogen der bereits genannten Alkanole und vor allem Diole ableiten; besonders bevorzugt sind Aethylendithio-Derivative. Zum Schutze der 3- sowie der 19-Oxogruppe sind Ketale und insbesondere Thioketale der oben angegebenen Art geeignet.

Die Ketalisierung oder Thioketalisierung, bzw. Acetalisierung oder Thioacetalisierung der zu schützenden Oxogruppen efolgt in einer an sich bekannten Weise, insbesondere unter den Bedingungen der Säurekatalyse und gegebenenfalls unter Anwendung von Wasser-entziehenden Mitteln bzw. der azeotropischen Destillation. Als Reagens verwendet man ülicherweise die obgenannten Alkohole bzw. Thiole als solche, oder aber als ihre reaktionsfähige Derivate, wie Acetale oder Ketale, bzw. Thioacetale oder Thioketale, insbesondere solche, in welchen die Carbonylkomponente leicht flüchtig ist, wie z.B. 2,2-Dimethyl-1,3-dioxolan bzw. 2,2-Dimethyl-1,3-dithiolan.

Die Abspaltung dieser Schutzgruppen erfolgt in an sich bekannter Weise durch Hydrolyse, vorzugsweise unter den allgemeinen Bedingungen der Säurekatalyse. Bei Thioketalen und Thioacetalen

arbeitet man vorzugsweise unter möglichst milden sauren Bedingungen, aber mit Zusatz einer Schwefel-bindenden Verbindung, z.B. eines Metallsalzes, insbesondere Schwermetallsalzes, wie Cadmiumcarbonat und/oder Quecksilber(II)chlorid. Da das letztgenannte Mittel selber in Anwesenheit von Wasser sauer reagiert, ist bei seiner Anwendung keine zusätzliche Säure als Katalysator notwendig.

Wenn zum vorübergehenden Schutz ein schwefelhaltiges Derivat angewandt wurde, ist es vorteilhaft, die Schutzgruppe noch vor der zweiten Verfahrensstufe zu entfernen, um Störungen im Verlaufe der Freisetzung der endständigen Aldehydgruppe zu vermeiden.

Wenn man eine Oxogruppe durch Umwandlung in eine Hydroxylgruppe vorübergehend schützt, verwendet man zu diesem Zwecke konventionelle Methoden, insbesondere Reduktion, z.B. mittels Diboran oder komplexer Hydride, wie Lithiumaluminiumhydrid oder Natriumborohydrid. Zur Rückbilding der Oxogruppe durch die nachträgliche Oxidation (bzw. Dehydrierung) der Hydroxylgruppe verwendet man auch konventionelle Methoden; als bevorzugte Oxidationsmittel gelten dabei Verbindungen des 6-wertigen Chroms, wie insbesondere Chromtrioxid, Chromsäure und ihre Alkalimetallsalze, wobei man als Reaktionsmedium vorteilhaft Niederalkancarbonsäuren, wie Essig- und Propionsäure, oder Pyridin oder Aceton, gegebenenfalls unter Verdünnung mit einem halogenierten Niederalkan, wie Dichlormethan oder Chloroform, verwendet und die Reaktionstemperatur vorzugsweise unterhalb der Raumtemperatur hält. Diese Art des Schutzes ist insbesondere für die 11-Oxogruppe geeignet, die dann während der Umsetzung als die 11$\alpha$- oder insbesondere die 11$\beta$-Hydroxylgruppe vorliegt, wobei sie schon vorher, z.B. während der Herstellung des Ausgangsstoffes, eingeführt werden kann.

Die zweite Stufe des erfindungsgemässen Verfahrens besteht in der Behandlung des erhaltenen Isomerengemisches aus der ersten Verfahrensstufe mit einem stark sauren hydrolysierenden Mittel, z.B. mit einer starken anorganischen sauerstoffhaltigen Säure, wie Phosphorsäure, Schwefelsäure oder Perchlorsäure, oder einer Halogenwasserstoffsäure, wie insbesondere der Chlorwasserstoffsäure, oder auch mit einer organischen Sulfonsäure, wie Methansulfonsäure, Benzolsulfonsäure, Toluolsulfonsäure, p-Brom- oder p-Chlorbenzolsulfonsäure. Die Umsetzung erfolgt jeweils in Anwesenheit von Wasser in einem konventionellem organischen Lösungsmittel, vorzugsweise einem mit Wasser mischbaren Lösungsmittel, oder Lösungsmittelgemisch, wie sie für die saure Hydrolyse üblich sind. Die konventionellen Entschwefelungsmittel, die man bei der Hydrolyse von Thioketalen üblicherweise verwendet, z.B. Cadmium- und Quecksilbersalze, sind bei dieser Behandlung nicht erforderlich.- Man arbeitet bei Temperaturen von etwa 0° bis etwa 100°, vorzugsweise bei Temperaturen um die Raumtemperatur; die Dauer der Reaktion bewegt sich in Abhängigkeit von jeweiligen Bedingungen (Temperatur, Säure- und Ausgangsstoffkonzentration, Lösungsmittel usw.) zwischen etwa 0,5—24 Stunden. Bei dieser sauren Behandlung werden zuerst die schwefelhaltigen 21-ständigen Substituenten der Seitenkette der Partialformel $—CH(OH)—CH(SCH_3)SOCH_3$ abgespalten und der $\alpha$-Hydroxyaldehyd der Partialformel $—CH(OH)—CH=O$ freigesetzt, dieser wird gleich unter den Reaktionsbedingungen, wahrscheinlich über ein Enol, zum gewünschten 21-Hydroxy-20-oxoendstoff der Partialformel $—CO—CH_2OH$ isomerisiert.

Gewünschtenfalls können die Endstoffe auch in Form entsprechender 21-Ester, d.h. nach einer anschliessenden konventionellen Veresterung (siehe unten), isoliert werden.

Im allgemeinen Kam des Verfahren Endstoffe ergeben die durch die allgemeine Formel

$$St(H)—CO—CH_2OH \qquad (I)$$

charakterisiert sind, worin St einen zweiwertigen Steroidrest der allgemeinen Formel

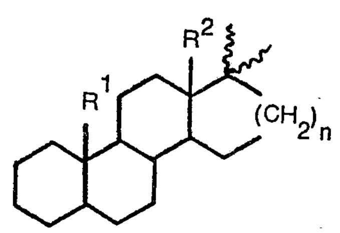

(St)

bedeutet, worin n die Zahl 1 oder 2, $R^1$ ein Wasserstoffatom, Methyl, ein freies, verestertes oder veräthertes Hydroxymethyl, ein freies oder funktionell abgewandeltes Formyl, oder ein freies oder verestertes Carboxyl, und $R^2$ einen gegebenenfalls halogenierten niederaliphatischen Kohlenwasserstoffrest mit 1—3 Kohlenstoffatomen, ein freies, verestertes oder veräthertes Hydroxymethyl, ein freies oder funktionell abgewandeltes Formyl, oder ein freies oder verestertes Carboxyl bedeutet, und worin in einer oder mehreren der Stellungen 1—16 Doppelbindungen, Halogenatome, Niederalkylreste, Methylenbrücken, freie oder funktionell abgewandelte Oxogruppen und/oder Oxidogruppen, sowie eine 3$\alpha$,5-transanulare einfache C—C-Bindung eines Cyclosteroids, einzeln oder in zweckmässigen Kombinationen vorhanden sein können. Die Wellenlinien in der oben gezeichneten Formel bedeuten insbesondere, dass die Seitenkette sowohl $\alpha$- wie auch $\beta$-orientiert sein kann. Doppelbindungen

befinden sich vorzugsweise in den Stellun- 1,2-, 4,5- 5,6-, 5,10-, 6,7- und/oder 9,11-, Hydroxylgruppen in der 3α-, 6β, 7α-, 11α-, 12α- und insbesondere 3β- und 11β-Stellung, Halogenatome, wie insbesondere Chlorung Fluoratome, in der 6α-, 9α- und 11β-Stellung, Niederalkylreste, insbesondere Methylreste, in den Stellungen 2α-, 6α-, 7α-, 16α- und 16β-, die Oxogruppe in der 3- und oder 11-Stellung, die Oxidogruppe in den Stellungen 5α,6α-, 5β,6β-, 6α,7α-, 9α,11α-, 11,18- und 6β,19-. Zu den besonders bevorzugten zweckmässigen Kombinationen der Substituenten in den Ringen A und B gehören z.B. eine freie oder veresterte 3β-Hydroxy-5-en- oder 3β-Hydroxy-6β,19-oxido-5α-Gruppierung, eine freie oder verätherte 3-Hydroxy-1,3,5(10)-trien- und 3α,5α-Cyclo-6β-hydroxy-Gruppierung, sowei eine freie, ketalisierte oder thioketalisierte 3-Oxo-4-en, und 3-Oxo-4,6-dien-Gruppierung. Zu besonders bevorzugten funktionellen Gruppen im Ring C, die sich mit denen der Ringe A und B weiter kombinieren, gehören in erster Linie die 9,11-Doppelbindung, die 9β,11β-Oxidogruppe und die 11β-Hydroxylgruppe; speziell zu nennen ist auch die 18 → 11β-Lacton-Bindung sowie die oxidische Brücke 11β,18 der Halbketal-Form der 18-Aldehydogruppe, wie sie z.B. im Aldosteron vorkommt.

Der Begriff "nieder", wo immer er im Zusammenhang mit einem organischen Rest vorkommt, bezeichnet einen entsprechenden Rest mit höchstens 7, vorzugsweise mit höchstens 4 Kohlenstoffatomen.

Vorzugsweise bedeutet das Symbol n die Zahl 1.

Ein Niederalkylrest ist z.B. ein n-Propyl-, i-Propyl-, n-Butyl-, i-Butyl-, sec-Butyl-, tert-Butyl-, ein verzweigter oder vorzugsweise gerader Pentyl-, Hexyl- oder Heptyl-Rest, vor allem aber ein Aethyl- oder Methyl-rest. Als ein niederaliphatischer Kohlenwasserstoffrest ist ein niederalkylrest, z.B. einer der bereits genannten, zu betrachten, der gegebenenfalls noch eine oder zwei mehrfache Bindungen, d.h. Doppelbindungen oder Acetylenbindungen, aufweist, wie z.B. ein Vinyl-, Allyl-, Propadienyl-, Propargyl- und Aethinylrest.

Der bereits charakterisierte niederaliphatische Kohlenwasserstoffrest bzw. Niederalkylrest kann durch einen oder mehrere Halogenatome, wie insbesondere Fluor- und/oder Chloratome, substituiert sein, wie z.B. Trifluormethyl-, Difluormethyl- oder 2-Chlorvinylrest.

Eine funktionell abgewandelte Oxogruppe oder Formylgruppe ist insbesondere eine ketalisierte bzw. acetalisierte oder thioketalisierte bzw. thioacetalisierte Oxo-bzw. Formylgruppe, z.B. eine solche, wie sie für den oben geschilderten vorübergehenden Schutz verwendet wird.

Eine verätherte Hydroxylgruppe kann eine Niederalkoxy-, insbesondere eine geradkettige Niederalkoxygruppe sein, z.B. die Methoxy-, Aethoxy-, Propoxy- und Butoxygruppe; vor allem ist sie aber eine durch eine leicht abspaltbare Schutzgruppe verätherte Hydroxylgruppe. Als die ätherartigen Schutzgruppen kommen die folgenden insbesondere in Betracht: ein durch Aryl, insbesondere Phenyl, in 1-Stellung substituierter Niederalkylrest, wie z.B. ein Benzyl- und Triphenylmethylrest; ein durch Niederalkoxygruppen, wie die obgenannten, in 1-Stellung substituierter Niederalkylrest, z.B. der 1-Butoxyäthyl- oder 1-Methoxyäthyl-rest; ferner heterocyclische Reste von Typ des 2-Tetrahydrofuryl- und insbesondere 2-Tetrahydropyranyl-restes; und schliesslich auch eine durch gleiche oder verschiedene Kohlenwasserstoffreste trisubstituierte Silylgruppe, insbesondere eine Triniederalkylsilylgruppe, z.B. die Trimethylsilyl- und Dimethyl-tert-butyl-silylgruppe.

Eine veresterte Hydroxylgruppe ist insbesondere eine solche, die durch eine Carbonsäure verestert ist; sie kann aber, als eine lactonisierte Hydroxylgruppe, durch eine im Molekül vorhandene Carboxylgruppe verestert sein.

Als Carbonsäure-Komponente einer veresterten Hydroxylgruppe kommen in erster Linie die in der Steroidchemie üblichen Carbonsäuren in Betracht, beispielsweise Monocarbonsäuren mit höchstens 18 Kohlenstoffatomen, wie aliphatische Carbonsäuren, insbesondere die Ameisensäure oder eine Niederalkancarbonsäure, deren Niederalkylrest einer der oben genannten ist, in erster Linie die Propion-, Butter-, Isobutter-, Valerian-, Isovalerian-, Oenanth- und Diäthylessigsäure und vor allem die Capron-, Trimethylessig- und Essigsäure; aber auch entsprechende halogenierte Niederalkancarbonsäuren, wie die Chloressigsäure, Trichlor- oder Trifluoressigsäure; sowie cycloaliphatische, cycloaliphatisch-aliphatische und aromatische Carbonsäuren, z.B. gegebenenfalls durch Halogen, wie Fluor, Chlor oder Brom, Hydroxy, Niederalkoxy, Niederalkyl und/oder Nitro substituierte Benzoesäuren oder entsprechende Aryl- oder Aryloxyniederalkancarbonsäuren, aber auch entsprechende Dicarbonsäuren mit höchstens 12 Kohlenstoffatomen, z.B. die Bernstein-, Glutar-, Adipin- und Phthalsäure.

Als veresterte Carboxylgruppe ist nicht nur eine Carboxylgruppe zu verstehen, die in Form ihres Esters mit einem Alkohol, insbesondere eines solchen mit einem Niederalkanol, vorliegt, sondern auch diejenige, welcher mit einer in einem geeigneten Abstand sich im Molekül befindenden Hydroxylgruppe einen 6- oder insbesondere 5-gliedrigen Lactonring schliesst.

Die erfindungsgemäss als Ausgangsstoffe verwendeten Steroidaldehyde sind durch die allgemeine Formel

$$St(H){-}CH=O \qquad (II)$$

charakterisiert, worin St die obgenannte Bedeutung hat. Sofern diese Ausgangsstoffe nicht bereits bekannt sind, sind sie durch bekannte allgemeine Verfahren zugänglich, z.B. durch die Umsetzung einer

entsprechenden 17- bzw. 17a-Oxoverbindung nach Wittig mit Methoxymethylentriphenylphosphoran oder einem analogen Reagens und durch die nachfolgende säure-katalysierte-hydrolyse der Intermediären 17- bzw. 17a-Methoxymethylen-Verbindung. (Dieses Verfahren ergibt jedoch nur das thermodynamisch stabile Isomere.)

In einer bevorzugten Durchführungsvariante des erfindungsgemässen Verfahrens werden Ausgangsstoffe verwendet, welche durch eine spezielle, besonders vorteilhafte Kombination von an sich bekannten Verfahren hergestellt wurden. Diese spezielle Ausgestaltung des erfindungsgemässen Verfahrens ist dadurch gekennzeichnet, dass man ein Keton der allgemeinen Formel

$$St=O \qquad (III)$$

worin St die oben genannte Bedeutung hat, zunächst durch Umsetzung mit Tosylmethylisocyanid (Ts-$CH_2$—N=C) in ein entsprechendes Cyanosteroid der allgemeinen Formel

$$St(H)—C\equiv N \qquad (IV)$$

worin St die obgenannte Bedeutung hat, umwandelt, dieses in an sich bekannter Weise, z.B. mit Diisobutylaluminiumhydrid oder einem analogen Reduktionsmittel, zum entsprechenden Steroidcarbaldehyd der oben charakterisierten allgemeinen Formel II reduziert und diesen anschliessend gemäss dem eingangs beschriebenen erfindungsgemässen Hauptverfahren in die Endstoffe der oben charakterisierten allgemeinen Formel I umwandelt. — Vorzugsweise werden alle gegebenenfalls im Molekül vorhandenen, an den Reaktionen nichtbeteiligten Oxogruppen während dieser Umsetzungen vorübergehend geschützt, vorzugsweise in der oben beim Hauptverfahren beschriebener Weise; es ist verteilhaft, wenn sich dieser Schutz nicht nur auf die Bildung der Cyano-verbindung und ihre Reduktion, sondern auch auf die anschliessende erfindungsgemässe Behandlung mit dem metallierten Reagens erstreckt.

Die Umsetzung der Oxoverbindung der Formel III mit Tosylmethylisonitril erfolgt in an sich bekannter Weise, insbesondere analog der im Tetrahedron *31*, 2151 und 2157 (1975) beschriebenen Methode, Als Produkt resultiert üblicherweise ein Gemisch vom $\alpha$- und $\beta$-Isomeren, die durch konventionelle physikalische Trennmethoden als individuelle Verbindungen isoliert werden können. Diese Herstellungsmethode ist insbesondere für die sonst sehr schwierig zugängliche $\alpha$-Reihe (d.h. die thermodynamisch weniger stabilen Isomeren)als ein allgemeines Verfahren vom grössten Interesse.

Die Reduktion der Cyanoverbindungen der Formel IV zu den Carbaldehyden der Formel II erfolgt auch in an sich bekannter Weise, insbesondere mit Diisobutylaluminiumhydrid der Formel $[(CH_3)_2CHCH_2)_2]AlH$ analog einer in J. Org. Chem. *29*, 3046 (1964) und J. Org. Chem. *35*, 858 (1970) beschriebenen Methode. Diese ist auch deshalb vorteilhaft, weil bei ihrer konventionellen Durchführung keine Epimerisierung eines weniger stabilen 17$\alpha$-Carbaldehyds in sein 17$\beta$-Isomeres stattfindet, obwohl eine solche Komplikation zu erwarten war.

Die Verfahrensprodukte der allgemeinen Formel I können nicht nur als solche, sondern gewünschtenfalls auch in Form ihrer 21-Ester, insbesondere Ester der eingangs hervorgehobenen in der Steroid-Chemie üblichen Carbonsäuren, durch nachträgliche konventionelle Acylierung der freien Hydroxylgruppen, wie insbesondere der 21-Hydroxylgruppe in primären Endstoffen der Formel I mit einer entsprechenden Säure oder ihrem reaktionsfähigen Derivat, wei insbesondere einem Anhydrid oder Halogenid, erhalten werden.

Unter anderem stellt das erfindungsgemässe Verfahren eine vorteilhafte Variante zur Herstellung von aldosteronantagonisierenden 19-oxygenierten 21-Hydroxyverbindungen der Pregnanreihe dar, welche in der Belgischen Patentschrift Nr. 867.634 beschrieben wurden.

Besonders vorteilhaft ist das erfindungsgemässe Verfahren für die Herstellung von Derivaten mit $\alpha$-orientierter Seitenkette, vor allem von Verbindungen der Formel

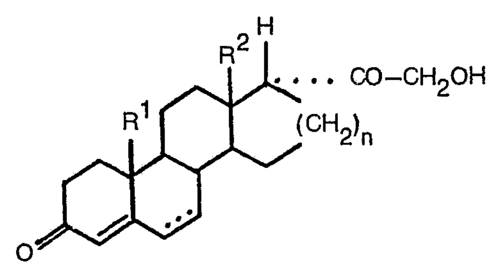

(IA)

worin n die Zahl 1 oder 2, $R^1$ Wasserstoffatom, Methyl, ein freies, verestertes oder veräthertes Hydroxymethyl, ein freies, acetalisiertes oder thioacetalisiertes Formyl, oder ein freies oder verestertes Carboxyl, und, $R^2$ einen gegebenenfalls halogenierten niederaliphatischen Kohlenwasserstoffrest mit 1—3

Kohlenstoffatomen, ein freies, verestertes oder veräthertes Hydroxymethyl, ein freies, acetalisiertes oder thioacetalisiertes Formyl, oder ein freies oder verestertes Carboxyl bedeutet, von entsprechenden 6,7-Dehydroderivaten und von 21-Estern dieser Verbindungen.

Unter den durch das erfindungsgemässe Verfahren erhältlichen Produkten sind Verbindungen der obigen Formel I (einschliesslich ihrer 6,7-Dehydroanalogen) hervorzuheben, worin $R^2$ Methyl oder Difluormethyl, und $R^1$ Hydroxymethyl, Methoxymethyl, Acetoxymethyl oder Wasserstoff und, wenn $n = 2$ und/oder $R^2$ Difluormethyl ist, auch Methyl bedeutet, und ganz speziell solche, worin n gleich 1 ist. Auch diese Verbindungen können als ihre 21-Ester, insbesondere die bereits hervorgehobenen, in erster Linie als Acetate, vorliegen.

Diese besonders hervorgehobenen Verbindungen können nicht nur als wertvolle Zwischenprodukte zur Herstellung von pharmakologisch verwendbaren Verbindungen, z.B. ihrer $\beta$-Isomeren, dienen, sondern weisen selber wertvolle physiologische Wirkungen, insbesondere als Agonisten und Antagonisten natürlicher Hormone von Warmblütern, insbesondere des Menschen, auf. So besitzt beispielsweise das 19,21-Dihydroxy-17$\alpha$-pregn-4-en-3,20-dion eine ausgeprägte antigestagene Wirksamkeit, wie es sich z.B. in modifiziertem Clauberg-McPhail-Test an mit Oestrogen und nachher mit Progesteron behandelten infantilen Kaninchen-Weibchen, insbesondere bei lokaler intrauteriner Applikation, zeigen lässt. Demgemäss sind sie in der hormonalen Therapie zur Regulierung des Spiegels von Sexualhormonen und Nebennierenrinde-Hormonen anwendbar. So ist z.B. die letzterwähnte Verbindung als solche oder in Form eines Esters, wie eines Monoacetats und insbesondere des Diacetats zur Unterbindung der Effekte des Progesterons und dadurch für Anticonception, vor allem als postcoitales Kontraceptivum, besonders geeignet. Analoge Eigenschaften und Anwendung findet man auch bei entsprechenden 6,7-Dehydroanalogen, in erster Linie bei 19,21-Dihydroxy-17$\alpha$-pregna-4,6-dien-3,20-dion.

Die vorliegende Erfindung, insbesondere im Zusammenhang mit dem Verfahren und seinen besonderen Ausgestaltungen, bezieht sich nicht nur auf Steroide der in der Natur vorhandenen bevorzugten D-Reihe, sondern auch auf die ensprechenden Antipoden der L-Reihe und racemische Gemische beider Antipoden, so wie in der Totalsynthese von Steroiden anfallen. In diesem Sinne ist der Begriff "Steroid" im ganzen Text zu verstehen.

Die Erfindung betrifft auch diejenigen Ausführungsformen des obigen Verfahren, bei denen man von einer auf irgendeiner Stufe als Zwischenprodukt erhältlichen Verbindung ausgeht und die fehlenden Schritte durchführt, oder bei denen ein Ausgangsstoff unter den Reaktionsbedingungen gebildet wird.

Die vorliegende Erfindung betrifft auch Präparate enthaltend mindestens eine neue Verbindung der Formel I oder der Formel IA, die zur Behandlung von Störungen der hormonalen Funktionen verschiedener Form verwendet werden. Sie enthalten eine wirksame Menge des Wirkstoffes allein oder im Gemisch mit anorganischen oder organischen, festen oder flüssigen, pharmazeutisch verwendbaren Trägerstoffen und, wenn erwünscht, auch mit anderen pharmakologisch bzw. therapeutisch wertvollen Stoffen und eignen sich insbesondere zu enteralen, z.B. oralen oder rektalen, oder zur parenteralen Verabreichung.

Unter dem Begriff "Wirkstoff" ist im ganzen nachfolgenden Text eine Verbindung der Formel I und insbesondere IA gemeint, wie sie oben durch die allgemeinen und insbesondere spezifischen Bedeutungen definiert ist.

Die vorliegende Erfindung betrifft insbesondere pharmazeutische Zusammensetzungen enthaltend als Wirkstoff mindestens eine der erfindungsgemässen Verbindungen der Formel IA (einschliesslich 6,7-Dehydroderivate und 21-Ester) in Form einer sterilen und/oder isotonischen wässrigen Lösung, oder aber im Gemisch mit mindestens einem festen oder halbfesten Trägerstoff.

Die vorliegende Erfindung betrifft auch Arzneimittel, sowie insbesondere Arzneimittel in Form von Dosierungseinheiten, welche mindestens eine der erfindungsgemässen Verbindungen allein oder im Gemisch mit einem oder mehreren Trägerstoffen enthalten, insbesondere solche in fester Form.

Die Erfindung betrifft insbesondere Arzneimittel in Form von Tabletten (einschliesslich Lutschtabletten, Granulen und Pastillen), Dragées, Kapseln, Pillen, Ampullen, Trockenvials oder Suppositorien enthaltend mindestens einen der genannten Wirkstoffe, allein oder im Gemisch mit einem oder mehreren Trägerstoffen.

Der Ausdruck "Arzneimittel" ist in dieser Beschreibung angewendet zur Bezeichnung von einzelnen abgetrennten Portionen einheitlicher Zusammensetzung, welche für die medizinische Verabreichung geeignet sind. Der Ausdruck "Arzneimittel in Form von Dosierungseinheiten" ist in dieser Beschreibung angewendet zur Bezeichnung von einzelnen abgetrennten Portionen einheitlicher Zusammensetzung, welche für die medizinische Verabreichung geeignet sind und je einzeln eine spezifische Menge des erfindungsgemässen Wirkstoffes enthalten, die etwa 0,025 bis etwa 4, vorzugsweise etwa 0,1 bis etwa 1 Tagesdosis entspricht.

Die Trägerstoffe zur Verwendung in den pharmazeutischen Zusammensetzungen (z.B. Granulaten) zur Herstellung von Tabletten, Dragées, Kapseln und Pillen sind z.B. die folgenden:

a) Verdünnungsmittel, z.B. Stärke, Zucker, wie Laktose, Glukose und Saccharose, Mannit, Sorbit und Kieselsäure,

b) Bindemittel, z.B. Carboxymethylcellulose und andere Cellulosederivate, Alginsäure und ihre Salze, wie Natriumalginat, Gelatine, und Polyvinylpyrrolidon,

c) Feuchtigkeitsregulatoren, z.B. Glycerin,

d) Sprengmittel, z.B. Agar-agar, Calciumcarbonat und Natriumbicarbonat,

e) Retardiermittel zur Verlangsamung der Aufnahme des Wirkstoffes, z.B. Paraffin,

f) Beschleuniger der Resorption, z.B. quaternäre Ammoniumverbindungen,

g) oberflächen-aktive Mittel, z.B. Cetylakohol und Glycerinmonoostearat,

h) Adsorptionsmittel, z.B. Kaolin und Bentonit,

i) Fliessregulier- und Schmiermittel, z.B. Talk, Calciumstearat, Magnesiumstearat und feste Polyäthylenglykole. Diese und ähnliche Träger- bzw. Hilfstoffe können auch mehreren der obgenannten Zwecke dienen.

Die Tabletten, Dragées, Kapseln und Pillen enthaltend die obgenannten erfindungsgemässen pharmazeutischen Zusammensetzungen können mit den üblichen Ueberzügen und Mantelmaterialien versehen werden, denen gewünschtenfalls Farbstoffe oder Pigmente, z.B. zur Identifizierungs- oder Kennzeichnungszwecken, beigemischt werden. Diese Ueberzüge können auch solche Zusammensetzung haben, welche eine verlangsamte Abgabe des Wirkstoffes ermöglicht; zu diesem Zwecke sind z.B. Wachse und Cellulosepräparate, wie Acetylcelulosephthalat oder Hydroxypropylmethylcelulosephthalat geeignet.

Diese Zusammensetzungen können auch in die Form dier Mikrokapseln verarbeitet werden.

Arzneimittel zur parenteralen Verabreichung sind vorzugsweise Ampullen enthaltend eine Einzeldosis des erfindungsgemässen Wirkstoffes, insbesondere eines wasserlöslichen physiologisch verträglichen Salzes, in Form einer wässrigen Lösung, welche vorzugsweise sterilisiert ist und gegebenenfalls die üblichen Puffermittel und/oder neutrale anorganische Salze, wie Natriumchlorid, als Hilfsmittel zur Einstellung der Isotonie mit Blut enthalten Eine solche wässrige Lösung eignet sich gut auch zur Herstellung von injizierbaren Feststoff-Arzneimittelformen, wie Trockenvials, in welchen die der Einzeldosis entsprechende Menge der Lösung in üblicher Weise, z.B. durch Lyophilisieren, abgedampft wird und der feste Rückstand erst unmittelbar vor dem Gebrauch mit sterilem Wasser in die Form der Injektionslösung gebracht wird.

Als Trägerstoffe für pharmazeutische Zusammensetzungen zur Verarbeitung zu Suppositorien eignen sich die üblichen Suppositorien-Grundmassenstoffe, z.B. natürliche oder synthetische Triglyceride, wie Kakaobutter, Paraffinkohlenwasserstoffe, Polyäthylenglykole und höhere Alkanole. Gelatine-Rektalkapseln enthalten als Grundmassenstoffe z.B. flüssige Triglyceride, Polyäthylenglykole oder Paraffinkohlenwasserstoffe.

Die erfindungsgemässen pharmazeutischen Zusammensetzungen enthalten vorzugsweise von etwa 0,1 bis zu etwa 99,5, insbesondere von etwa 1 bis zu etwa 90 Gewichts-% des Wirkstoffes.

Die empfohlene Tagesdosis für einen 75 kg schweren Warmblüter beträgt etwa 5—500 mg, vorzugsweise etwa 20—300 mg, sie kann jedoch innerhalb weiten Grenzen in Abhängigkeit von Species, Alter und der individuellen Ansprechbarkeit varieren.

Die Herstellung der obgenannten erfindungsgemässen pharmazeutischen Zusammensetzungen, Präparate, Arzneimitteln und Arzneimitteln in Form von Dosierungseinheiten erfolgt mittels konventioneller, an sich bekannter Herstellungsverfahren der pharmazeutischen Industrie, z.B. mittels üblicher Misch-, Granulier-, Tablettier-, Dragier-, Lösungs- und Lyophilisierungsverfahren, wobei gewünschtenfalls unter keimfreien Bedingungen gearbeitet wird oder ein Zwischenprodukt oder ein fertiges Produkt sterilisiert wird.

Die vorliegende Erfindung betrifft auch die Anwendung der erfindungsgemässen Wirkstoffe zur Bekämpfung von verschiedenen Formen der hormonalen, insbesondere sexualhormonalen Störungen im Mensch und anderen Warmblütern, sowie eine entsprechende therapeutische Methode, die durch die Verabreichung einer wirksamen Dosis mindestens eines der erfindungsgemässen Wirkstoffe allein oder zusammen mit einem oder mehreren Trägerstoffen oder in einer Arzneimittelform charakterisiert ist. Die erfindungsgemässen Wirkstoffe werden dabei enteral, z.B. oral, rektal, vaginal oder intrauterin, oder parenteral, wie intraperitoneal oder intravenös, verabreicht.

In den folgenden Beispielen, welche die Erfindung weiter illustrieren, ohne sie dabei einzuschränken, sind die Temperaturen in Celsiusgraden angegeben.

## Beispiel 1

a) Unter Argon-Atmosphäre wird aus 34 g Kalium und 980 ml tert-Butanol eine Lösung von Kalium-tert-butoxid zubereitet; dieser wird unter Rühren eine Lösung von 45 g 3,3-Aethylendithio-19-hydroxy-androsta-4,6-dien-17-on in 1000 ml 1,2-Dimethoxyäthan bei Raumtemperatur unter Argon-Atmosphäre schnell zugetropft. Nach 15-minutigem Rühren wird das Reaktionsgemisch innert 90 Minuten bei 25° mit einer Lösung von 35,5 g Tosylmethyl-isocyanid in 1000 ml 1,2-Dimethoxyäthan versetzt, noch eine weitere Stunde gerührt, und auf Eis-Wasser gegossen. Die organische Schicht wird abgetrennt, die wässrige mit Methylenchlorid extrahiert, und die vereinigten organischen Auszüge werden mit Wasser gewaschen, über Natriumsulfat getrocknet und im Wasserstrahlvakuum eingedampft. Der Rückstand wird über eine Kieselgelsäule chromatographiert; mit einem Gemisch von Hexan-Aethylacetat (4:1) eluiert man 24 g 3,3-Aethylendithio-17β-cyano-androsta-4,6-dien-19-ol,

Smp. 180—181° nach einfacher Kristallisation aus Methylenchlorid-Diisopropyläther; $[\alpha]_D = +162°$ (c = 0,14; Chloroform). Weiteres Eluieren mit demselben Lösungsmittelgemisch ergibt 12 g 3,3-Aethylendithio-17α-cyano-androsta-4,6-dien-19-ol, Smp. 211—213° (aus Methylenchlorid-Aethylacetat); $[\alpha]_D = +61°$ (c = 0,48; Chloroform).

b) Eine Lösung von 24 g 3,3-Aethylendithio-17β-cyanoandrosta-4,6-dien-19-ol in 450 ml 1,2-Dimethoxyäthan wird bei −20° tropfenweise innert 15 Minuten mit 400 ml einer 20-prozentigen Lösung von Diisobutylaluminiumhydrid in Toluol unter Rühren versetzt, auf 25° erwärmen gelassen und bei dieser Temperatur noch eine weitere Stunde gerührt. Das Reaktionsgemisch wird auf Eis-Wasser gegossen, mit Salzsäure angesäuert und eine Stunde gerührt. Das Produkt wird in Methylenchlorid aufgenommen, die organische Phase nacheinander mit Wasser, wässriger Natriumhydrogencarbonat-Lösung und Wasser gewaschen, über Natriumsulfat getrocknet und im Wasserstrahlvakuum eingedampft. Der Rückstand wird über eine Kieselgel-Säule chromatographiert; durch Eluieren mit einem Gemisch Toluol-Aethylacetat (95:5) erhält man 3,3-Aethylendithio-19-hydroxy-androsta-4,6-dien-17β-carbaldehyd, Smp. 165—166° (aus Methylenchlorid-Diisopropyläther), $[\alpha]_D = +198°$ (c = 0,474, Chloroform).

c) Eine Lösung von 6,7 ml Formaldehyd-dimethylthioacetal-S-oxid (Methylthiomethyl-methylsulfoxid) in 80 ml Tetrahydrofuran wird unter Argon-Atmosphäre bei −20° mit 13,5 ml einer 1,6-molaren Lösung von Butyllithium in Hexan tropfenweise behandelt, so dass die Temperatur nicht über −17° aufsteigt. Anschlissend wird das Reaktionsgemisch mit einer Lösung von 13 g 3,3-Aethylendithio-19-hydroxy-androsta-4,6-dien-17β-carbaldehyd in 100 ml Tetrahydrofuran innert 30 Minuten tropfenweise versetzt und weitere 30 Minuten gerührt. Das Reaktionsgemisch wird auf Eis-Wasser gegossen und das Produkt in Aethylacetat aufgenommen. Die vereinigten organischen Auszüge werden nacheinander mit Wasser und einer gesättigten wässrigen Natriumchloridlösung gewaschen, über Natriumsulfat getrocknet, im Wasserstrahlvakuum eingedampft, und der Rückstand auf eine Kieselgelsäule aufgetragen. Durch Eluieren mit einem Gemisch von Hexan-Aethylacetat (1:1) wird das unumgesetzte Ausgangsmaterial regeneriert; mit einem Gemisch von Aethylacetat-Aceton (2:1) eluiert man Fraktionen, die nach Abdampfen ein kristallines Isomerengemisch von 3,3-Aethylendithio-21ξ-methylsulfinyl-21ξ-methylthio-pregna-4,6-dien-19,20ξ-diol, das ohne Trennung weiterverarbeitet wird.

d) Eine Lösung von 15,9 g des gemäss c) erhältlichen Isomerengemisches in 960 ml Aceton wird mit 42 ml Wasser, 12 g Quecksilber(II)chlorid und 12 g Cadmiumcarbonat versetzt, 5 Stunden bei Zimmertemperatur gerührt und durch eine Schicht von Kieselguhr abgenutscht. Der Filterkuchen wird mit Methylenchlorid extrahiert, der Auszug mit dem ursprünglichen Filtrat vereinigt, und eingedampft. Das resultierende rohe Gemisch von isomeren 19,20ξ-Dihydroxy-21ξ-methylsulfinyl-21ξ-methylthio-pregna-4,6-dien-3-onen wird direkt in der nächsten Stufe weiterverarbeitet.

e) Das Isomerengemisch von Stufe d) wird in 300 ml Tetrahydrofuran gelöst, mit 50 ml 5N-Salzsäure versetzt und 13 Stunden bei Zimmertemperatur gerührt. Das Reaktionsgemisch wird auf 2 Liter Eis-Wasser gegossen und das Produkt in Methylenchlorid aufgenommen. Die vereinigten Auszüge werden nacheinander mit einer verdünnten Natriumcarbonat-Lösung, Wasser und einer gesättigten Natriumchlorid-Lösung gewaschen, über Natriumsulfat getrocknet und im Wasserstrahlvakuum eingedampft. Der Rückstand wird über eine Kieselgelsäule chromatographiert; durch Eluieren mit einem Gemisch von Hexan-Aceton (2:1) erhält man 19,21-Dihydroxy-pregna-4,6-dien-3,20-dion, das nach Kristallisation aus Aceton-Hexan bei 163—165° schmilzt.

In analoger Weise wird das 17α-Cyano-Epimere (siehe Stufe a) gemäss den Stufen b-e verarbeitet, womit das 19,21-Dihydroxy-17α-pregna-4,6-dien-3,20-dion, Smp. 192—195° (aus Chloroform-Aethylacetat) resultiert.

Beispiel 2

In analoger Weise wie im Beispiel 1 beschrieben ergibt das 3,3-Aethylendithio-19-hydroxy-androst-4-en-17-on ein Isomerengemisch, woraus man das 3,3-Aethylendithio-17β-cyano-androst-4-en-19-ol, Smp. 205—206°, und das isomere 3,3-Aethylendithio-17α-cyano-androst-4-en-19-ol, Smp. 171—172°, isoliert. Das letztere Isomere wird zum amorphen 3,3-Aethylendithio-19-hydroxy-androst-4-en-17α-carbaldehyd reduziert, dieser in das Isomerengemisch von 3,3-Aethylendithio-21ξ-methylsulfinyl-21ξ-methylthio-17α-pregn-4-en-19,20ξ-diol umwandelt, das in zwei Stufen zum 19,21-Dihydroxy-17α-pregn-4-en-3,20-dion, Smp. 207—212° hydrolysiert wird. Das entsprechende 19,21-Diacetat schmilzt, aus Methylenchlorid-Methanol umkristallisiert, bei 124—125°.

Das 17β-Nitril ergibt über analoge Zwischenprodukte der β-Reihe 19,21-Dihydroxy-pregn-4-en-3,20-dion, Smp. (146°) 163—168°; das entsprechende 19,21-Diacetat Schmilzt bei 124—125° (mit Depression bei Misch-schmelzpunkt mit dem 17α-Epimeren).

Beispiel 3

In analoger Weise wie im Beispiel 1 beschrieben ergibt das 3,3-Aethylendithio-18,18-difluor-androst-4-en-17-on ein Gemisch bestehend aus 3,3-Aethylendithio-18,18-difluor-17β-cyano-androst-4-en, Smp. 145—146°, und 3,3-Aethylendithio-18,18-difluor-17α-cyano-androst-4-en, Smp. 230—232°. Das rohe Produkt wird zum Gemisch vom 3,3-Aethylendithio-18,18-difluor-androst-4-en-

17β-carbaldehyde und -17α-carbaldehyd reduziert. Dieses Gemisch wird gemäss den Verfahrensstufen c, d und e des Beispiels 1 verarbeitet und das erhaltene Isomerengemisch in das 21-Hydroxy-18,18-difluor-17α-pregn-4-en-3,20-dion, Smp. 165—167°, und das 21-Hydroxy-18,18-difluor-pregn-4-en-3,20-dion, Smp. 140—141°, aufgetrennt. Alternativ werden beide isolierten 17-Cyanoverbindungen separat verarbeitet.

Beispiel 4

In analoger Weise wie im Beispiel 1 beschrieben ergibt das 3,3-Aethylendithio-19-methoxy-androst-4-en-17-on ein Isomerengemisch, woraus man das 3,3-Aethylendithio-17β-cyano-19-methoxy-androst-4-en, Smp. 173—174°, isoliert. Dieses wird zum amorphen 3,3-Aethylendithio-19-methoxyandrost-4-en-17β-carbaldehyd reduziert und gemäss den Verfahrensstufen c-e des Beispiels 1 weiterverarbeitet, womit das 21-Hydroxy-19-methoxypregn-4-en-3,20-dion, Smp. 148—149°, resultiert.

Beispiel 5

In analoger Weise wie im Beispiel 1 beschrieben ergibt das 3,3-Aethylendithio-7α-methyl-östr-4-en-17-on das 3,3-Aethylendithio-17β-cyano-7α-methyl-östr-4-en, Smp. 231—232°, welches zum 3,3-Aethylendithio-7α-methyl-östr-4-en-17β-carbaldehyd reduziert und danach gemäss den Verfahrensstufen c-e des Beispiels 1 weiterverarbeitet wird. Das resultierende 21-Hydroxy-7α-methyl-19-norpregn-4-en-3,20-dion schmilzt bei 160°C.

Beispiel 6

In analoger Weise wie im Beispiel 1 werden die folgenden Endstoffe ausgehend von entsprechenden 17-Oxoverbindungen erhalten:

a) 21-Hydroxy-pregna-4,6-dien-3,20-dion, charakterisiert als 21-Acetat, Smp. 113—114°;
b) 21-Hydroxy-pregna-1,4,6-trien-3,20-dion, charakterisiert als 21-Acetat, Smp. 170—172°;
c) 21-Hydroxy-pregna-4,6,9(11)-trien-3,20-dion, charakterisiert als 21-Acetat, Smp. 133—134°;
d) 21-Hydroxy-2,2-dimethyl-pregn-4-en-3,20-dion, Smp. 118—120°;
e) 21-Hydroxy-16α-methyl-pregn-4-en-3,20-dion, charakterisiert als 21-Acetat, Smp. 132°;
f) 9α-Fluor-11β,21-dihydroxy-16α-methyl-pregna-1,4-dien-3,20-dion, Smp. 216—222°;
g) 21-Hydroxy-19-nor-pregn-4-en-3,20-dion, Smp. 136—137°;
h) 16α-Aethyl-21-hydroxy-19-nor-pregn-4-en-3,20-dion;
i) 19,21-Dihydroxy-pregna-4,6-dien-3,20-dion-19-acetat, Smp. 105—107°;
j) 21-Hydroxy-3,20-dioxo-pregna-1,4-dien-18-al, charakterisiert als 21-Acetat, Smp. 158—160°;
k) 21-Hydroxy-3,20-dioxo-pregna-4,6-dien-18-al, charakterisiert als 21-Acetat, Smp. 121—122°;
l) 18,21-Dihydroxy-pregna-4,6-dien-3,20-dion, Smp. 170—172°, 21-Acetat, Smp. 143—144°;
m) 21-Hydroxy-3,20-dioxo-pregn-4-en-19-al, Smp. 162—164°;
n) 21-Hydroxy-3,20-dioxo-pregna-4,6-dien-19-al, Smp. 164—166°;
o) 21-Hydroxy-19-methoxy-pregna-4,6-dien-3,20-dion, charakterisiert als 21-Acetat, Smp. 158—165°;
p) 3,21-Dihydroxy-19-nor-pregna-1,3,5(10)-trien-20-on, Smp. 190°——191°.
q) 3β,21-Dihydroxy-17α-methyl-D-homo-pregn-5-en-20-on, Smp. 198—202°; und
r) 11β,21-Dihydroxy-17α-methyl-D-homo-pregn-4-en-3,20-dion, Smp. 198—200°.

**Patentansprüche für die Vertragsstaaten: BE CH DE FR GB IT NL SE**

1. Verfahren zum Aufbau der Hydroxyacetyl-Seitenkette von Steroiden des Pregnan-Typs einschliesslich der D-Homo-verbindungen, dadurch gekennzeichnet, dass man einen entsprechenden Steroidcarbaldehyd unter vorübergehendem Schutz übriger gegebenenfalls vorhandener Oxogruppen mit Formaldehyd-dimethylmercaptal-S-oxid in Form eines Alkalimetallsalzes davon, und danach, gewünschtenfalls nach Regenierung der übrigen Oxogruppen, mit einem stark sauren hydrolysierenden Mittel behandelt und gewünschtenfalls einen erhaltenen Endstoff acyliert.

2. Verfahren gemäss Anspruch 1, dadurch gekennzeichnet, dass man von einem Steroidcarbaldehyd mit geschützten übrigen, gegebenenfalls im Steroidrest vorhandenen Oxogruppen ausgeht, welcher in einer an sich bekannter Weise durch die Umsetzung eines entsprechenden 17- bzw. 17a-Ketosteroids, dessen übrige, gegebenenfalls vorhandene Oxogruppen in einer geschützten Form vorliegen, mit Tosylmethyl-isocyanid und die nachfolgende Reduktion des entsprechenden 17- bzw. 17a-Cyanosteroids hergestellt wird.

3. Verfahren gemäss Anspruch 1 oder 2, dadurch gekennzeichnet, dass man eine Verbindung der Formel

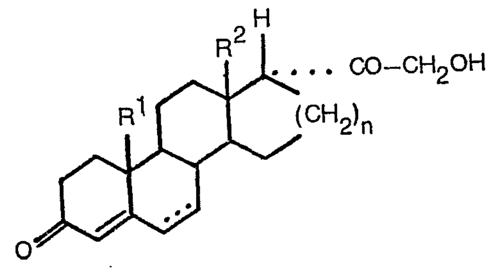

(IA)

worin n die Zahl 1 oder 2, $R^1$ Wasserstoffatom, Methyl, ein freies, verestertes oder veräthertes Hydroxymethyl, ein freies, acetalisiertes oder thioacetalisiertes Formyl, oder ein freies oder verestertes Carboxyl, und $R^2$ einen gegebenenfalls halogenierten niederaliphatischen Kohlenwasserstoffrest mit 1—3 Kohlenstoffatomen, ein freies, verestertes oder veräthertes Hydroxymethyl, ein freies, acetalisiertes oder thioacetalisiertes Formyl, oder ein freies oder verestertes Carboxyl bedeutet, ein entsprechendes 6,7-Dehydroderivat oder ein 21-Ester dieser Verbindungen, herstellt.

4. Eine Verbindung der Formel IA, worin n die Zahl 1 oder 2, $R^2$ Methyl oder Difluormethyl, und $R^1$ Hydroxymethyl, Methoxymethyl, Acetoxymethyl oder Wasserstoff und, wenn $n = 2$ und/oder $R^2$ Difluormethyl ist, auch Methyl bedeutet, ein entsprechendes 6,7-Dehydroderivat und ein 21-Ester dieser Verbindungen.

5. Eine Verbindung der Formel IA, worin n die Zahl 1, $R^1$ Hydroxymethyl oder Acetoxymethyl und $R^2$ Methyl bedeutet, ein entsprechendes 6,7-Dehydroderivat und ein 21-Acetat dieser Verbindungen.

6. 19,21-Dihydroxy-17$\alpha$-pregn-4-en-3,20-dion.

7. 19,21-Dihydroxy-17$\alpha$-pregn-4-en-3,20-dion-diacetat.

8. 19,21-Dihydroxy-17$\alpha$-pregna-4,6-dien-3,20-dion.

9. Pharmazeutische Präparate enthaltend eine der Verbindungen der Ansprüche 4—8.

10. Anticonceptionsmittel enthaltend eine der Verbindungen der Ansprüche 5—8.

**Patentansprüche für den Vertragsstaat: AT**

1. Verfahren zum Aufbau der Hydroxyacetyl-Seitenkette von Steroiden des Pregnan-Typs einschliesslich der D-Homo-verbindungen, dadurch gekennzeichnet, dass man einen entsprechenden Steroidcarbaldehyd unter vorübergehendem Schutz übriger gegebenenfalls vorhandener Oxogruppen mit Formaldehyd-dimethylmercaptal-S-oxid in Form eines Alkalimetallsalzes davon, und danach, gewünschtenfalls nach Regenerierung der übrigen Oxogruppen, mit einem stark sauren hydrolysierenden Mittel behandelt und, gewünschtenfalls einen erhaltenen Endstoff acyliert.

2. Verfahren gemäss Anspruch 1, dadurch gekennzeichnet, dass man von einem Steroidcarbaldehyd mit geschützten übrigen, gegebenenfalls im Steroidrest vorhandenen Oxogruppen ausgeht, welcher durch die Umsetzung eines entsprechenden 17- bzw. 17a-Ketosteroids, dessen übrige, gegebenenfalls vorhandene Oxogruppen in einer geschützten Form vorliegen, mit Tosylmethylisocyanid und die nachfolgende Reduktion des entsprechenden 17- bzw. 17a-Cyanosteroids hergestellt wird.

3. Verfahren gemäss Anspruch 1 oder 2, dadurch gekennzeichnet, dass man eine Verbindung der Formel

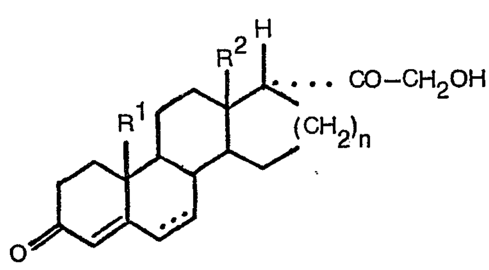

(IA)

worin n die Zahl 1 oder 2, $R^1$ Wasserstoffatom, Methyl, ein freies, verestertes oder veräthertes Hydroxymethyl, ein freies oder funktionell abgewandeltes Formyl, oder ein freies oder verestertes Carboxyl, und $R^2$ einen gegebenenfalls halogenierten niederaliphatischen Kohlenwasserstoffrest mit 1—3 Kohlenstoffatomen, ein freies, verestertes oder veräthertes Hydroxymethyl, ein freies oder funktionell abgewandeltes Formyl, oder ein freies oder verestertes Carboxyl bedeutet, ein entsprechendes 6,7-Dehydroderivat oder einen 21-Ester dieser Verbindungen herstellt, wobei in einer Verbindung, in welcher $n = 1$, nur eines der Symbole $R^1$ und $R^2$ für Methyl stehen kann.

4. Verfahren gemäss Anspruch 1 oder 2, dadurch gekennzeichnet, dass man eine Verbindung der Formel IA, worin n die Zahl 1 oder 2, $R^2$ Methyl oder Difluormethyl, und $R^1$ Hydroxymethyl, Methoxymethyl, Acetoxymethyl oder Wasserstoff und, wenn $n = 2$ und/oder $R^2$ Difluormethyl ist, auch Methyl bedeutet, ein entsprechendes 6,7-Dehydroderivat oder einen 21-Ester dieser Verbindungen herstellt.

5. Verfahren gemäss Anspruch 1 oder 2, dadurch gekennzeichnet, dass man eine Verbindung der Formel IA, worin n die Zahl 1, $R^1$ Hydroxymethyl oder Acetoxymethyl und $R^2$ Methyl bedeutet, ein entsprechendes 6,7-Dehydroderivat oder das 21-Acetat dieser Verbindungen herstellt.

6. Verfahren gemäss Anspruch 1 oder 2, dadurch gekennzeichnet, dass man 19,21-Dihydroxy-17$\alpha$-pregn-4-en-3,20-dion herstellt.

7. Verfahren gemäss Anspruch 1 oder 2, dadurch gekennzeichnet, dass man 19,21-Dihydroxy-17$\alpha$-pregn-4-en-3,20-dion-diacetat herstellt.

8. Verfahren gemäss Anspruch 1 oder 2, dadurch gekennzeichnet, dass man 19,21-Dihydroxy-17$\alpha$-pregna-4,6-dien-3,20-dion herstellt.

**Claims for the Contracting States: BE CH DE FR GB IT NL SE**

1. A process for synthesising the hydroxyacetal side-chain of steroids of the pregnane type including the D-homo-compounds, which process comprises treating a corresponding steroid carbaldehyde, while temporarily protecting any other oxo groups present, with formaldehyde dimethylmercaptal-S-oxide in the form of an alkali metal salt thereof, and subsequently, if desired after regeneration of the other oxo groups, with a strongly acid hydrolysing agent and, if desired, acylating a resultant final product.

2. A process according to claim 1, wherein the starting material is a steroid carbaldehyde in which any other oxo groups present in the steroid radical are in protected form, which steroid carbaldehyde is prepared in a manner known per se by reaction of a corresponding 17- or 17$\alpha$-ketosteroid in which any other oxo groups present are in protected form, with tosylmethylisocyanide and subsequent reduction of the corresponding 17- or 17$\alpha$-cyanosteroid.

3. A process according to either of claims 1 or 2, for the preparation of a compound of the formula

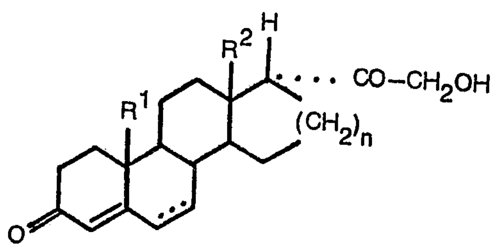

(IA)

wherein n is 1 or 2, $R^1$ represents a hydrogen atom, methyl, a free, esterified or etherified hydroxymethyl group, a free, acetylated or thioacetylated formyl group, or a free or esterified carboxyl group, and $R^2$ represents an unsubstituted or a halogenated lower aliphatic hydrocarbon radical of 1 to 3 carbon atoms, a free, esterified or etherified hydroxymethyl group, a free, acetylated or thioacetylated formyl group, or a free or esterified carboxyl group, a corresponding 6,7-dehydro derivative or a 21-ester thereof.

4. A compound of the formula IA, wherein n is 1 or 2, $R^2$ represents methyl or difluoromethyl, and $R^1$ represents hydroxymethyl, methoxymethyl, acetoxymethyl or hydrogen, and, if n is 2 and/or $R^2$ is difluoromethyl, $R^1$ also represents methyl, and a corresponding 6,7-dehydro derivative and a 21-ester thereof.

5. A compound of the formula IA, wherein n is 1, $R^1$ represents hydroxymethyl or acetoxymethyl and $R^2$ represents methyl, and a corresponding 6,7-dehydro derivative and a 21-acetate thereof.

6. 19,21-Dihydroxy-17$\alpha$-pregn-4-ene-3,20-dione.

7. 19,21-Dihydroxy-17$\alpha$-pregn-4-ene-3,20-dione diacetate.

8. 19,21-Dihydroxy-17$\alpha$-pregna-4,6-diene-3,20-dione.

9. A pharmaceutical preparation containing a compound as claimed in any one of claims 4 to 8.

10. A contraceptive agent containing a compound as claimed in any one of claims 5 to 8.

**Claims for the Contracting State: AT**

1. A process for synthesising the hydroxyacetal side-chain of steroids of the pregnane type including the D-homo-compounds, which process comprises treating a corresponding steroid carbaldehyde, while temporarily protecting any other oxo groups present, with formaldehyde dimethylmercaptal-S-oxide in the form of an alkali metal salt thereof, and subsequently, if desired after regeneration of the other oxo groups, with a strongly acid hydrolysing agent and, if desired, acylating a resultant final product.

2. A process according to claim 1, wherein the starting material is a steroid carbaldehyde in which any other oxo groups present in the steroid radical are in protected form, which steroid carbaldehyde is

prepared in a manner known per se by reaction of a corresponding 17- or 17$\alpha$-ketosteroid in which any other oxo groups present are in protected form, with toxylmethylisocyanide and subsequent reduction of the corresponding 17- or 17$\alpha$-cyanosteroid.

3. A process according to either of claims 1 or 2, for the preparation of a compound of the formula

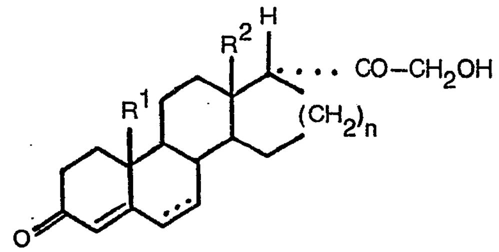

(IA)

wherein n is 1 or 2, $R^1$ represents a hydrogen atom, methyl, a free, esterified or etherified hydroxymethyl group, a free or functionally modified formyl group, or a free or esterified carboxyl group, and $R^2$ represents an unsubstituted or a halogenated lower aliphatic hydrocarbon radical of 1 to 3 carbon atoms, a free, esterified or etherified hydroxymethyl group, a free or functionally modified formyl group, or a free or esterified carboxyl group, a corresponding 6,7-dehydro derivative or a 21-ester thereof with the proviso that, in a compound in which n is 1, only one of the symbols $R^1$ and $R^2$ may represent methyl.

4. A process according to either of claims 1 or 2 for the preparation of a compound of the formula IA, wherein n is 1 or 2, $R^2$ represents methyl or difluoromethyl, and $R^1$ represents hydroxymethyl, methoxymethyl, acetoxymethyl or hydrogen, and, if n is 2 and/or $R^2$ is difluoromethyl, $R^1$ also represents methyl, and a corresponding 6,7-dehydro derivative or a 21-ester thereof.

5. A process according to either of claims 1 or 2 for the preparation of a compound of the formula IA, wherein n is 1, $R^1$ represents hydroxymethyl or acetoxymethyl and $R^2$ represents methyl, and a corresponding 6,7-dehydro derivative or a 21-acetate thereof.

6. A process according to either of claims 1 or 2 for the preparation of 19,21-dihydroxy-17$\alpha$-pregn-4-ene-3,20-dione.

7. A process according to either of claims 1 or 2 for the preparation of 19,21-dihydroxy-17$\alpha$-pregn-4-ene-3,20-dione diacetate.

8. A process according to either of claims 1 or 2 for the preparation of 19,21-dihydroxy-17$\alpha$-pregna-4,6-diene-3,20-dione.

**Revendications pour les Etats contractants: BE CH DE FR GB IT NL SE**

1. Procédé de synthèse de la chaîne latérale hydroxyacétyle de stéroïdes du type pregnanique y compris les composés D-homo, caractérisé en ce qu'on traite un carbaldéhyde stéroïdique correspondant avec protection temporaire d'autres groupes oxo éventuellement présents avec du formaldéhyde-diméthylmercaptal-S-oxyde sous la forme d'un de ses sels de métaux alcalins, puis, éventuellement après régénération des autres groupes oxo, avec un agent hydrolysant acide fort et si on le désire en ce qu'on acyle un produit final obtenu.

2. Procédé selon la revendication 1, caractérisé en ce qu'on part d'un carbaldéhyde stéroïdique ayant d'autres groupes oxo, éventuellement présents dans le radical stéroïde, protégés, et que l'on prépare de façon classique par la réaction d'un 17- ou 17a-cétostéroïde correspondant, dont les autres groupes oxo éventuellement présents se présentent sous forme protégée, avec de l'isocyanure de tosylméthyle puis par réduction du 176 cu 17a-cyanostéroïde correspondant.

3. Procédé selon l'une des revendications 1 ou 2, caractérisé en ce qu'on prépare un composé de formule

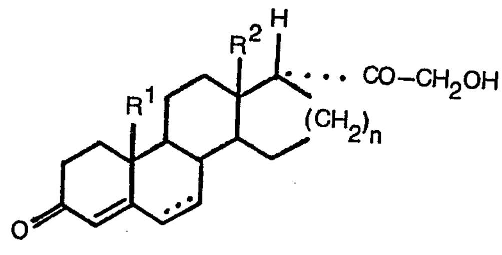

(IA)

où n représente le nombre 1 ou 2, $R^1$ un atome d'hydrogène, un méthyle, un hydroxyméthyle libre, estérifié ou éthérifié, un formyle libre, acétalisé ou thioacétalisé, ou un carboxyle libre ou estérifié, et $R^2$

un radical hydrocarboné aliphatique inférieur éventuellement halogéné en $C_1$ à $C_3$, un hydroxyméthyle libre, estérifié ou éthérifié, un formyle libre, acétalisé ou thioacétalisé où un carboxyle libre ou estérifié, un dérivé 6,7-déshydro correspondant ou un 21-ester de ces composés.

4. Composé de formule IA où n représente le nombre 1 ou 2, $R^2$ un méthyle ou un difluorométhyle, et $R^1$ un hydroxyméthyle, un méthoxyméthyle, un acétoxyméthyle ou un hydrogène et également, si n = 2 et/ou $R^2$ est un difluorométhyle, un méthyle, un dérivé 6,7-déshydro correspondant et un 21-ester de ces composés.

5. Composé de formule IA, où n représente le nombre 1, $R^1$ un hydroxyméthyle ou un acétoxyméthyle et $R^2$ un méthyle, un dérivé 6,7-déshydro correspondant et un 21-acétate de ces composés.

6. 19,21-dihydroxy-17$\alpha$-pregn-4-èn-3,20-dione.

7. 19,21-dihydroxy-17$\alpha$-pregn-4-èn-3,20-dione-diacétate.

8. 19,21-dihydroxy-17$\alpha$-pregna-4,6-dién-3,20-dione.

9. Préparations pharmaceutique contenant l'un des composés des revendications 4—8.

10. Agent anticonceptionnel contenant l'un des composés des revendications 5—8.

**Revendications pour l'Etat contractant: AT**

1. Procédé de synthèse de la chaîne latérale hydroxyacétyle de stéroïdes du type pregnanique, y compris les composés D-homo, caractérisé en ce qu'on traite un carbaldéhyde stéroïdique correspondant avec protection temporaire d'autres groupes oxo éventuellement présents, avec du formaldéhyde-diméthylmercaptal-S-oxyde sous la forme d'un de ses sels de métaux alcalins, puis, éventuellement après régénération des autres groupes oxo, avec un agent hydrolysant fortement acide et, si on le désire, en ce qu'on acyle un produit final obtenu.

2. Procédé selon la revendication 1, caractérisé en ce qu'on part d'un carbaldéhyde stéroïdique ayant d'autres groupes oxo éventuellement présents dans le radical stéroïdique proté gés, que l'on prépare par la réaction d'un 17- ou 17a-cétostéroïde correspondant, dont les autres groupes oxo éventuellement présents se présentent sous forme protégée, avec de l'isocyanure de tosylméthyle puis par réduction du 17- ou 17a-cyanostéroïde correspondant.

3. Procédé selon l'une des revendications 1 ou 2, caractérisé en ce qu'on prépare un composé de formule

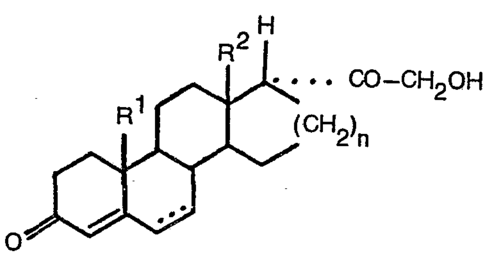

(IA)

où n représente le nombre 1 ou 2, $R^1$ un atome d'hydrogène, numéthyle, un hydroxyméthyle libre, estérifié ou éthérifié, un formyle libre ou fonctionnellement modifié, ou un carboxyle libre ou estérifié, et $R^2$ représente un radical hydrocarboné aliphatique inférieur éventuellement halogéné en $C_1$ à $C_3$, un hydroxyméthyle libre, estérifié ou éthérifié, un formyle libre ou fonctionnellement modifié, ou un carboxyle libre ou estérifié, un dérivé 6,7-déshydro correspondant ou un 21-ester de ces composés, où dans un composé où n égal 1, seul l'un des symboles $R^1$ et $R^2$ peut représenter un méthyle.

4. Procédé selon l'une des revendications 1 ou 2, caractérisé en ce qu'on prépare un composé de formule IA, où n représente le nombre 1 ou 2, $R^2$ un méthyle ou un difluorométhyle, et $R^1$ un hydroxyméthyle, un méthoxyméthyle, un acétoxyméthyle ou un hydrogène et également, si n = 2 et/ou $R^2$ est un difluorométhyle, un méthyle, un dérivé 6,7-déshydro correspondant ou un 21-ester de ces composés.

5. Procédé selon l'une des revendications 1 ou 2, caractérisé en ce qu'on prépare un composé de formule IA, où n représente le nombre 1, $R^1$ un hydroxyméthyle ou un acétoxyméthyle et $R^2$ un méthyle, un dérivé 6,7-déshydro correspondant ou le 21-acétate de ces composés.

6. Procédé selon l'une des revendications 1 ou 2, caractérisé en ce qu'on prépare la 19,21-dihydroxy-17$\alpha$-pregn-4-èn-3,20-dione.

7. Procédé selon l'une des revendications 1 ou 2, caractérisé en ce qu'on prépare le 19,21-dihydroxy-17$\alpha$-pregn-4-èn-3,20-dione-diacétate.

8. Procédé selon l'une des revendications 1 ou 2, caractérisé en ce qu'on prépare la 19,21-dihydroxy-17$\alpha$-pregna-4,6-dién-3,20-dione.